(19)

Europäisches
Patentamt
European
Patent Office
Office européen
des brevets

(11) **EP 4 567 423 A1**

(12) **EUROPEAN PATENT APPLICATION**

(43) Date of publication:
**11.06.2025 Bulletin 2025/24**

(21) Application number: **24213199.3**

(22) Date of filing: **15.11.2024**

(51) International Patent Classification (IPC):
***G01N 33/00*** (2006.01)      *A01B 69/04* (2006.01)
*A01M 1/02* (2006.01)

(52) Cooperative Patent Classification (CPC):
**G01N 33/0098**; A01B 69/008; A01M 1/026

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **06.12.2023  IN 202321083027**

(71) Applicant: **Tata Consultancy Services Limited
Maharashtra (IN)**

(72) Inventors:
• **MOHITE, JAYANTRAO
400601 Thane (West), Maharashtra (IN)**

• **LONKAR, VAIBHAV SADASHIV
London, EC3R7BB (GB)**
• **SINGH, DINESHKUMAR JANG BAHADUR
400601 Thane (West) , Maharashtra (IN)**
• **SAWANT, SURYAKANT ASHOK
411057 Pune, Maharashtra (IN)**
• **RAJPOOT, NANDAN SINGH
453112 Indore, Madhya Pradesh (IN)**
• **SARANGI, SANAT
400601 Thane (West),  Maharashtra (IN)**
• **PAPPULA, SRINIVASU
500034 Hyderabad, Telangana (IN)**

(74) Representative: **Goddar, Heinz J.
Boehmert & Boehmert
Anwaltspartnerschaft mbB
Pettenkoferstrasse 22
80336 München (DE)**

(54) **ESTIMATION OF EFFECTIVE PEST-DISEASE SEVERITY SCORE USING A PERSONALIZED CROP PROTOCOL DRIVEN UNMANNED SCOUTING VEHICLE**

(57)     Precise estimation of effective pest-disease severity is a challenge due to their ability of quick spread, resistance to pesticide development, quality, and systematic data. Conventionally, an inter-dependency of sequence of operations and impact of the inter-dependency on data collection has not been considered. Embodiments of the present disclosure provide a system and method for estimation of effective pest-disease severity score using a personalized crop protocol driven unmanned scouting vehicle. The system of the present disclosure intelligently makes use of the personalized crop protocol and feedback from users on a farm for determining optimal sampling locations from the farm. Further, a standardized and systematic way of optimal data capture from precise plant positions in the farm is provided using selected sensors at appropriate time. The standardized and systematic way of optimal data capture considers the impact of various pests, disease, and natural enemies available at a given instance.

**FIG. 6B**

EP 4 567 423 A1

**Description**

CROSS-REFERENCE TO RELATED APPLICATIONS AND PRIORITY

The present application claims priority to Indian application no. 202321083027, filed on 06 December 2023.

TECHNICAL FIELD

The disclosure herein generally relates to the field of estimation of effective pest-disease severity score, and, more particularly, to system and method for estimation of effective pest-disease severity score using a personalized crop protocol driven unmanned scouting vehicle.

BACKGROUND

In agriculture field, with abrupt and erratic changes in weather, crops are prone to multiple pest, diseases and other variety of stresses such as water, nutrient deficiency, and/or the like. Changing weather patterns and increased temperatures can alter pest and disease behavior and distribution, making forecasting and management strategies more challenging. Specifically, for the pest or the diseases, there are various indirect and direct approaches for monitoring and forecasting. Indirect approaches mainly focus on indirect indicators or parameters such as weather, soil, and/or the like and use them with actual count of pest observed for monitoring and forecasting. However, direct approaches involve techniques such as visual scoring, image analysis, spectral imaging, remote sensing, and/or the like. Data collected from all of these techniques have been basically used to analyze the pest or the disease detection, severity estimation.

There exist several approaches that have mainly focused on collecting the data in their own way and then analyzing the data for specific objective. However, collecting data from precise locations at the right time is crucial and challenging. There were certain approaches involving the use of remote sensing data for detection of locations and the path for field data collection. However, those approaches do not consider other ancillary factors and metadata associated with farm. Moreover, various routing algorithms have been developed for agricultural data collection which mainly consider a variability derived from historical datasets on soil, weather, historical and current data on remote sensing, and/or the like. While the routing algorithms are useful for general data collection, these are not optimal when targeting specific pest or diseases.

Moreover, due to availability of various on-field, human and remote sensors, extensive data is being collected directly or indirectly at repeated frequencies. While data quantity is increasing, there is a need to ensure collection of accurate and comprehensive data at precise locations, using selected sensors and at right time. Further, precise estimation of effective pest-disease severity is still a challenge due to their ability of quick spread, resistance to pesticide development, quality, systematic data, and/or the like.

SUMMARY

Embodiments of the present disclosure present technological improvements as solutions to one or more of the above-mentioned technical problems recognized by the inventors in conventional systems. For example, in one embodiment, a processor implemented method is provided. The processor implemented method, comprising acquiring, via one or more hardware processors, a plurality of data pertaining to a farm in a specific region; deriving, via the one or more hardware processors, a personalized crop protocol specific to the farm in the specific region using the plurality of data, wherein the personalized crop protocol captures dynamic interplay of one or more factors affecting a crop grown in the farm and suggests a precise sequence of practices required to be followed; determining, via the one or more hardware processors, a plurality of optimal sampling locations on the farm in the specific region using the personalized crop protocol specific to the farm, wherein the plurality of optimal sampling locations are determined based on a plurality of hotspot scores, and wherein each of the plurality of optimal sampling locations represents at least one of (i) a pest hotspot, (ii) a disease hotspot and (iii) a natural enemies hotspot; generating, via the one or more hardware processors, a vehicle routing path protocol for an unmanned scouting vehicle based on the plurality of optimal sampling locations, a layout of the farm, information on potential growth of a plurality of plants projected by the personalized crop protocol and a plurality of available resources, using a reinforcement learning technique, wherein the vehicle routing path protocol represents an optimal path required to be traversed by the unmanned scouting vehicle to reach each of the plurality of optimal sampling locations; collecting, via the one or more hardware processors, a plurality of phenotyping characteristics of a plant at each plant position from a plurality of plant positions identified at each optimal sampling location from the plurality of optimal sampling locations on the optimal path traversed by the unmanned scouting vehicle, wherein the plurality of phenotyping characteristics of the plant are collected using a multi-degree-of-freedom mechanism on the unmanned scouting vehicle that adjusts one or more sensor positions with high precision allowing multi-angle data capture from a plurality of plant parts at a specific time instance; computing, via the one or more hardware processors, a risk score for at least one of (i) the pest and (ii) the disease at the specific time instance at each plant position from the plurality of plant positions by processing the collected plurality of phenotyping characteristics of the plant using one or more models; computing, via the one or more hardware processors, an aggregated plant risk score for the at least one of (i) the pest and (ii) the disease at the specific time instance in each plant from a plurality of plants with respect to crop stage, wherein the aggregated plant risk score is the sum of the risk scores and a weightage associated with the at least one of (i) the pest and (ii) the disease at each plant position from the plurality of plant positions; and estimating, via the one or more hardware processors, an effective pest-disease severity score for the farm based on a sum of the aggregated risk score for the at least one of (i) the pest and (ii) the disease at the specific time instance in each plant from a plurality of plants with respect to crop stage.

In another aspect, a system is provided. The system comprising a memory storing instructions; one or more communication interfaces; an unmanned scouting vehicle; and one or more hardware processors coupled to the memory via the one or more communication interfaces, wherein the one or more hardware processors are configured by the instructions to: acquire a plurality of data pertaining to a farm in a specific region; derive a personalized crop protocol specific to the farm in the specific region using the plurality of data, wherein the personalized crop protocol captures dynamic interplay of one or more factors affecting a crop grown in the farm and suggests a precise sequence of practices required to be followed; determine a plurality of optimal sampling location on the farm in the specific region using the personalized crop protocol specific to the farm , wherein the plurality of optimal sampling locations are determined based on a plurality of hotspot scores, and wherein each of the plurality of optimal sampling locations represents at least one of (i) a pest hotspot, (ii) a disease hotspot and (iii) a natural enemies hotspot; generate a vehicle routing path protocol for an unmanned scouting vehicle based on the plurality of optimal sampling locations, a layout of the farm, information on potential growth of a plurality of plants projected by the personalized crop protocol and a plurality of available resources, using a reinforcement learning technique, wherein the vehicle routing path protocol represents an optimal path required to be traversed by the unmanned scouting vehicle to reach each of the plurality of optimal sampling locations; collect a plurality of phenotyping characteristics of a plant at each plant position from a plurality of plant positions identified at each optimal sampling location from the plurality of optimal sampling locations on the optimal path reversed by the unmanned scouting vehicle, wherein the plurality of phenotyping characteristics of the plant are collected using a multi-degree-of-freedom mechanism on the unmanned scouting vehicle that adjusts one or more sensor positions with high precision allowing multi-angle data capture from a plurality of plant parts at a specific time instance; compute a risk score for at least one of (i) the pest and (ii) the disease at the specific time instance at each plant position from the plurality of plant positions by processing the collected plurality of phenotyping characteristics of the plant using one or more models; compute an aggregated plant risk score for the at least one of (i) a pest and (ii) a disease at the specific time instance in each plant from a plurality of plants with respect to crop stage, wherein the aggregated plant risk score is the sum of the risk scores and a weightage associated with the at least one of (i) the pest and (ii) the disease at each plant position from the plurality of plant positions; and estimate an effective pest-disease severity score for the farm based on a sum of the aggregated risk score for the at least one of (i) the pest and (ii) the disease at the specific time instance in each plant from a plurality of plants with respect to crop stage.

In yet another aspect, a non-transitory computer readable medium is provided. The non-transitory computer readable medium are configured by instructions for acquiring a plurality of data pertaining to a farm in a specific region; deriving, via the one or more hardware processors, a personalized crop protocol specific to the farm in the specific region using the plurality of data, wherein the personalized crop protocol captures dynamic interplay of one or more factors affecting a crop grown in the farm and suggests a precise sequence of practices required to be followed; determining a plurality of optimal sampling locations on the farm in the specific region using the personalized crop protocol specific to the farm, wherein the plurality of optimal sampling locations are determined based on a plurality of hotspot scores, and wherein each of the plurality of optimal sampling locations represents at least one of (i) a pest hotspot, (ii) a disease hotspot and (iii) a natural enemies hotspot; generating a vehicle routing path protocol for an unmanned scouting vehicle based on the plurality of optimal sampling locations, a layout of the farm, information on potential growth of a plurality of plants projected by the personalized crop protocol and a plurality of available resources, using a reinforcement learning technique, wherein the vehicle routing path protocol represents an optimal path required to be traversed by the unmanned scouting vehicle to reach each of the plurality of optimal sampling locations; collecting a plurality of phenotyping characteristics of a plant at each plant position from a plurality of plant positions identified at each optimal sampling location from the plurality of optimal sampling locations on the optimal path traversed by the unmanned scouting vehicle, wherein the plurality of phenotyping characteristics of the plant are collected using a multi-degree-of-freedom mechanism on the unmanned scouting vehicle that adjusts one or more sensor positions with high precision allowing multi-angle data capture from a plurality of plant parts at a specific time instance; computing a risk score for at least one of (i) the pest and (ii) the disease at the specific time instance at each plant position from the plurality of plant positions by processing the collected plurality of phenotyping characteristics of the plant using one or more models; computing an aggregated plant risk score for the at least one of (i) the pest and (ii) the disease at the specific time instance in each plant from a plurality of plants with respect to crop stage, wherein the aggregated plant risk score is the sum of the risk scores and a weightage associated with the at least one of (i) the pest and (ii) the disease at each plant position from the plurality of plant positions; and estimating an effective pest-disease severity score for the farm based on a sum of the aggregated risk score for the at least one of (i) the pest and (ii) the disease at the specific time instance in each plant from a plurality of plants with respect to crop stage.

In accordance with an embodiment of the present disclosure, the one or more hardware processors are configured to the plurality of optimal sampling locations are dynamically updated based on (i) one or more changes in the personalized crop protocol, (ii) a plurality of user inputs received on the farm and (iii) a plurality of real-time sensor data acquired from the unmanned scouting vehicle.

In accordance with an embodiment of the present disclosure, the plurality of plant positions are dynamically identified using the personalized crop protocol and a knowledge graph.

In accordance with an embodiment of the present disclosure, the unmanned scouting vehicle possesses reinforcement learning ability.

In accordance with an embodiment of the present disclosure, the unmanned scouting vehicle is equipped with data processing at edge to refrain from sharing redundant data to a data server.

It is to be understood that both the foregoing general description and the following detailed description are exemplary and explanatory only and are not restrictive of the invention, as claimed.

BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary system for estimation of effective pest-disease severity score using a personalized crop protocol driven unmanned scouting vehicle, according to some embodiments of the present disclosure.

FIGS. 2A and 2B illustrate an exemplary flow diagram illustrating a method for estimation of effective pest-disease severity score using a personalized crop protocol driven unmanned scouting vehicle, in accordance with some embodiments of the present disclosure.

FIG. 3 shows a frontal view of a farm in a specific region illustrating process of acquiring data for estimation of effective pest-disease severity score using a personalized crop protocol driven unmanned scouting vehicle, in accordance with some embodiments of the present disclosure.

FIGS. 4A and 4B shows a top view of the farm illustrating identification of the plurality of hotspots for estimation of effective pest-disease severity score using a personalized crop protocol driven unmanned scouting vehicle, in accordance with some embodiments of the present disclosure.

FIG. 5 shows a top view of the farm illustrating the process of generation of the vehicle routing protocol for an unmanned scouting vehicle, in accordance with some embodiments of the present disclosure.

FIGS. 6A and 6B show a top view of the farm illustrating standardized and systematic way of data capture from precise plant positions using selected sensors and at the right time, in accordance with some embodiments of the present disclosure.

FIG. 7 shows a view of a handheld device for providing real-time user feedback, in accordance with some embodiments of the present disclosure.

DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments. It is intended that the following detailed description be considered as exemplary only, with the true scope being indicated by the following embodiments described herein.

Precise estimation of effective pest-disease severity is a challenge due to their ability of quick spread, resistance to pesticide development, quality, and systematic data. There is a need to consider a standardized and systematic approach of data collection guided by personalized crop protocol for the field while developing routing protocols and optimal locations for the farm.

Conventional approaches have focused on historical and current datasets from weather, soil, remote sensing to estimate variability and use of the same for deciding optimal locations. Such conventional approaches do not consider inter-dependency of sequence of operations and impact of that on data collection. Further, conventional approaches are limited to optimal locations from the farm but did not consider a combination of specific position of plant, specific part of plant to be scanned to get an optimal result depending on the age, stage, type, and/or the like of plant, positions of the plant, multiple sensors mounted on the vehicle with respect to crop protocol for the field at a given instance or crop stage for precise data collection. Consideration of multiple pests and natural enemies available in the field at a given instance and their relevance (weighting) to specific crop stage/age has not been considered for estimating precise effective pest-disease severity score for the farm.

The present disclosure addresses the unresolved problem of the conventional approaches by providing a system and method for estimation of effective pest-disease severity score using a personalized crop protocol driven unmanned scouting vehicle. The key objective is to intelligently make use of the personalized crop protocol and feedback from users on a farm for deciding optimal sampling locations from the farm. Embodiments of the present disclosure provide a system and method for standardized and systematic way of data capture from the field by integrated use of specific position of plant, specific part of plant to be scanned to get an optimal result depending on the age, stage, type, etc. of plant, multiple sensors mounted on the vehicle with respect to crop protocol for the field at a given instance/crop stage for precise data collection. Further, an approach of quantitative and precise estimation of effective pest-disease severity score is provided by considering impact of various pests and natural enemies available at a given instance with respect to the crop stage. More specifically, the present disclosure describes the following:

1. An integrated system and method for optimal sampling location generation using personalized crop protocol guided routing protocol for precision data collection utilizing unmanned scouting vehicle equipped with multiple sensors.
2. A system and method for standardized and optimal data capture from precise plant positions using selected sensors at appropriate time.
3. A system and method for estimation of effective pest and/or disease severity score for a farm at a given time.

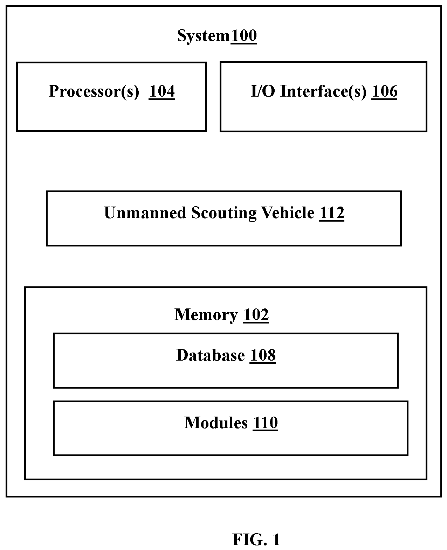

Referring now to the drawings, and more particularly to FIGS. 1 through 7, where similar reference characters denote corresponding features consistently throughout the figures, there are shown preferred embodiments and these embodiments are described in the context of the following exemplary system and/or method.

FIG. 1 illustrates an exemplary system for estimation of effective pest-disease severity score using a personalized crop protocol driven unmanned scouting vehicle, according to some embodiments of the present disclosure. In an embodiment, the system 100 includes or is otherwise in communication with one or more hardware processors 104, communication interface device(s) or input/output (I/O) interface(s) 106, one or more data storage devices or memory 102 operatively coupled to the one or more hardware processors 104, and an unmanned scouting vehicle 112 such as autonomous drone, rover, autonomous robots or other aerial and ground vehicles, and/or the like. The one or more hardware processors 104, the memory 102, and the I/O interface(s) 106 may be coupled to a system bus or a similar mechanism 110. The system 100, with one or more hardware processors present across the physically distributed components, is configured to execute functions of one or more functional blocks or components (servers) of the system 100. In an embodiment, the servers may be used to process requests and send responses, to execute an algorithm for data fusion, reinforcement learning, image processing, and/or the like. The algorithm for data fusion is used to integrate data from multiple sensors for comprehensive insights and estimation of effective pest-disease severity score for the farm. The algorithm for reinforcement learning is used to learn from current and past data collection to optimize on future data collection approach dynamically. The algorithm for image processing is used for analyzing captured images and extracting plant pest, disease, and natural enemies information.

Referring to the components of system 100, in an embodiment, the I/O interface(s) 106 may include a variety of software and hardware interfaces, for example, a web interface, a graphical user interface, a GIS software, and the like. The GIS software is used to overlay data, create maps, and visualize hotspots. The I/O interface(s) 106 may include a variety of software and hardware interfaces, for example, interfaces for peripheral device(s), such as a keyboard, a mouse, an external memory, a plurality of sensor devices, a printer and the like. Further, the I/O interface(s) 106 may enable the system 100 to communicate with other devices, such as web servers and external databases. The plurality of sensor devices may include environmental sensors to determine temperature, humidity, light intensity, soil moisture, pH, and/or the like, light sensors to determine appropriate timing for data capture, imaging sensors which includes high-resolution cameras for capturing images of precise plant positions, videos, spectrometers that includes hyperspectral or multispectral sensors, and sound sensors for capturing ultrasound.

The I/O interface(s) 106 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface(s) 106 may include one or more ports for connecting a number of computing systems with one another or to another server computer. Further, the I/O interface (s) 106 can include one or more ports for connecting to a number of external devices or to another server or devices. The external devices can include a GPS Module and positioning System with sensing, processing, communicating, displaying and storage capability, external third party data sources for accurate positioning and navigation of the unmanned scouting vehicle, precise plant position recording, and for various types of information associated with estimation of effective pest-disease severity score using a personalized crop protocol driven unmanned scouting vehicle.

The one or more hardware processors 104 may be implemented as one or more microprocessors, microcomputers, microcontrollers, digital signal processors, central processing units, state machines, logic circuitries, and/or any devices that manipulate signals based on operational instructions. Among other capabilities, the one or more hardware processors 104 are configured to fetch and execute computer-readable instructions stored in the memory 102. In the context of the present disclosure, the expressions 'processors' and 'hardware processors' may be used interchangeably. In an embodiment, the system 100 can be implemented in a variety of computing systems, such as laptop computers, portable computer, notebooks, hand-held devices, workstations, mainframe computers, servers, a network cloud and the like.

The memory 102 may include any computer-readable medium known in the art including, for example, volatile memory, such as static random access memory (SRAM) and dynamic random access memory (DRAM), and/or non-volatile memory, such as read only memory (ROM), erasable programmable ROM, flash memories, hard disks, optical disks, and magnetic tapes.

In an embodiment, the memory 102 includes a plurality of modules 110 distributed across each of the distributed components (servers). The plurality of modules 110 include programs or coded instructions that supplement applications or functions performed by the components of system 100 for executing different steps involved in the process of optimal sampling location generation using personalized crop protocol and routing protocol driven unmanned scouting vehicle equipped with multiple sensors for precision data collection, and estimation of effective pest-disease severity score for a farm at a given time, being performed by the system 100. The plurality of modules 110, amongst other things, can include routines, programs, objects, components, and data structures, which performs particular tasks or implement particular abstract data types. The plurality of modules 110 may also be used as, signal processor(s), node machine(s), logic circuitries, and/or any other device or component that manipulates signals based on operational instructions. Further, the plurality of modules 110 can be used by hardware, by computer-readable instructions executed by the one or more hardware processors 104, or by a combination thereof. The plurality of modules 110 can include various sub-modules (not shown).

Further, the memory 102 may comprise information pertaining to input(s)/output(s) of each step performed by the processor(s) 104 of the system 100 and methods of the present disclosure. Further, the memory 102 includes a database 108, which can be a distributed storage for each of the components such as servers. The database (or repository) 108 may include one or more models and data that is processed, received, or generated as a result of the execution of the one or more models and the plurality of modules in the module(s) 110. The one or more models may include one or more machine learning models for predicting optimal sampling locations based on historical data, one or more pest life cycle models which are mathematical models to predict pest behavior and infestation cycles, pest-natural enemy interaction models to estimate the impact of natural enemies on pest severity, and/or the like.

The database stores a plurality of historical data, a plurality of real time data, a plurality of crop specific data, information pertaining to pest and disease, a plurality of geospatial data, a plurality of sensor data, a plurality of pathfinding and navigation data, and one or more user inputs and annotations for the farm, and/or the like. The plurality of historical data includes historical pest and disease incidence records, previous crops grown, crop maturity days, crop yields and growth patterns, and past weather data to identify trends and seasonal variations, and/or the like. The plurality of real time data includes current weather conditions including temperature, humidity, precipitation, and wind speed, soil moisture levels, soil composition, real time data from environmental sensors deployed in the farm, real-time imagery from cameras or drones, and/or the like. The plurality of crop specific data includes crop growth models and growth stage data, plant physiological data such as photosynthesis rates and transpiration levels, and information about specific crop varieties being cultivated. The information pertaining to pest and disease includes pest and disease species, life cycle and phenotype data, data on natural enemies and their effectiveness in controlling pests, and pest and disease identification information. The plurality of geospatial data includes field boundaries and topographical data, Geographic Information System (GIS) data for spatial analysis, and satellite imagery for monitoring large-scale changes. The plurality of sensor data includes data from various sensors including cameras, spectrometers, environmental sensors, sound sensors, sensor data on temperature, humidity, light intensity, soil conditions, and/or the like. The plurality of pathfinding and navigation data includes geographic coordinates for mapping and navigation, topological maps for efficient route planning, and obstacle detection and avoidance data for pathfinding. The one or more user inputs and annotations include inputs from agronomists, farmers, and experts, annotations and labels for training machine learning models.

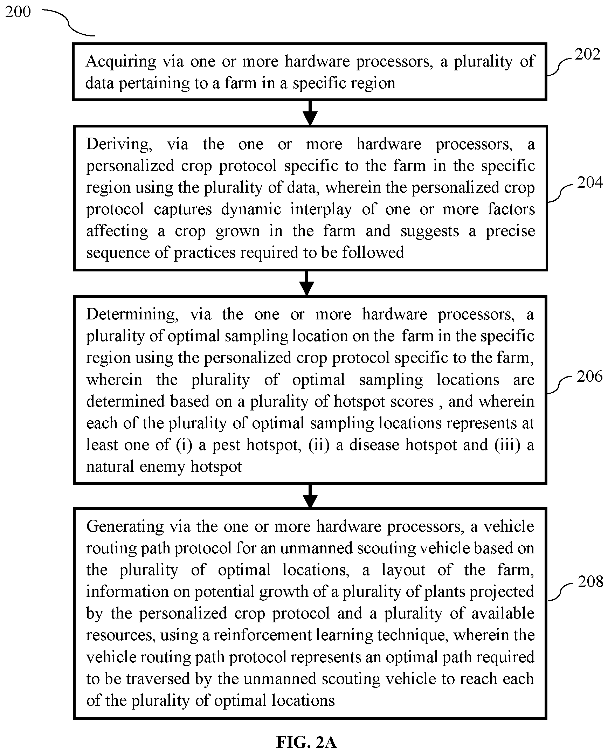

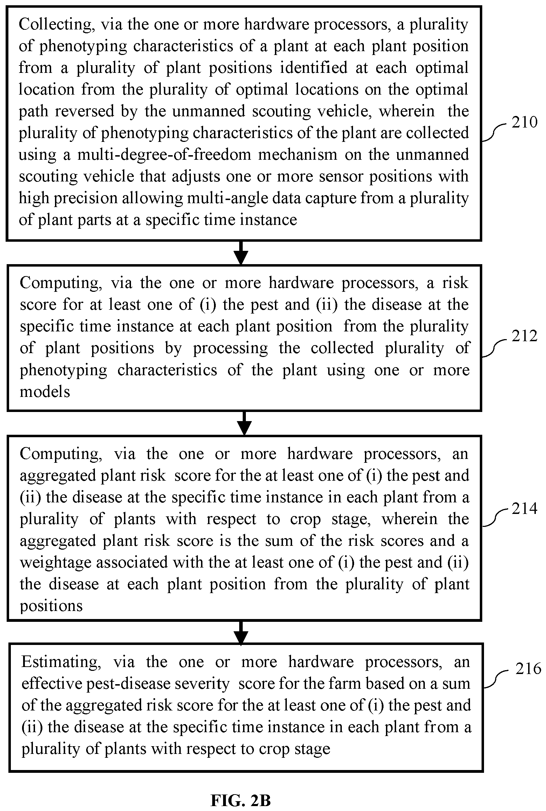

Although the database 108 is shown internal to the system 100, it will be noted that, in alternate embodiments, the database 108 can also be implemented external to the system 100, and communicatively coupled to the system 100. The data contained within such an external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS) or any non-relational database such as NoSQL or document databases. Functions of the components of the system 100 are now explained with reference to architecture as depicted in steps in flow diagrams in FIGS. 2A and 2B.

FIGS. 2A and 2B, with reference to FIG. 1, illustrate an exemplary flow diagram illustrating a method for estimation of effective pest-disease severity score using a personalized crop protocol driven unmanned scouting vehicle, using the system 100 of FIG. 1, in accordance with some embodiments of the present disclosure. Referring to FIG. 1, in an embodiment, the system(s) 100 comprises one or more data storage devices or the memory 102 operatively coupled to the one or more hardware processors 104 and is configured to store instructions for execution of steps of the method by the one or more processors 104. The steps of the method 200 of the present disclosure will now be explained with reference to components of the system 100 of FIG. 1, the flow diagram as depicted in FIGS. 2A and 2B, and one or more examples. Although steps of the method 200 including process steps, method steps, techniques or the like may be described in a sequential order, such processes, methods, and techniques may be configured to work in alternate orders. In other words, any sequence or order of steps that may be described does not necessarily indicate a requirement that the steps be performed in that order. The steps of processes described herein may be performed in any practical order. Further, some steps may be performed simultaneously, or some steps may be performed alone or independently.

Referring to FIG. 2A, at step 202 of the present disclosure, one or more hardware processors are configured to acquire a plurality of data pertaining to a farm in a specific region. In the context of present disclosure, the expressions 'farm', 'field', and 'land' can be interchangeably used throughout the description.

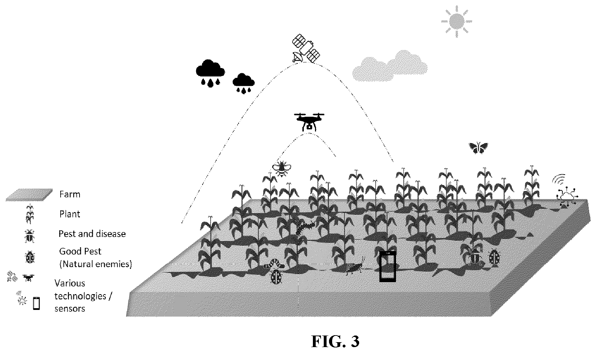

In an embodiment, the plurality of data may comprise but are not limited to hyper-localized data including crop stages, actual and forecasted weather, soil conditions, remote sensing data, on-field sensor readings, high-resolution satellite imagery, and historical pest/disease/natural enemy records, and/or the like. FIG. 3 shows a frontal view of a farm in a specific region illustrating process of acquiring data for estimation of effective pest-disease severity score using a personalized crop protocol driven unmanned scouting vehicle, in accordance with some embodiments of the present disclosure. The farm shown in FIG. 3 is a smart farm which is equipped with good seed variety, source of irrigation, sunlight, and different types of sensing elements such as satellite, drone, cell phone app, IoT, communication network, and/or the like. This can help divide the one or more farms in the specific region in different "sensing zones". Technologies helps in capturing micro and macro data continuously from the farm and accordingly generate a potential risk severity score for each sensing zone. But more granular data is required to be created for each sensing zone to ensure that management practices are sustainable and there is neither overutilization of resources, nor any emissions.

Further, at step 204 of the present disclosure, the one or more hardware processors 104? are configured to derive a personalized crop protocol specific to the farm in the specific region using the plurality of data. The personalized crop protocol is derived using the plurality of data considering hyper-localized data on crop stages, weather (actual and forecast), soil, remote sensing, on-field sensors, high resolution satellite data, historical situation of pest/diseases/natural enemies in the specific region. The personalized crop protocol captures dynamic interplay of one or more factors affecting a crop grown in the farm and suggests a precise sequence of practices required to be followed. In other words, the personalized crop protocol is a sequence of farm operations that needs to be performed on the farm. This personalized crop protocol is dynamically updated based on recommendation, feedback or data acquired from one or more sensors, models, and/or the like. The one or more factors may include but are not limited to growth stage, weather conditions, crop variety, soil characteristics, agro-climatic zone, and historical vulnerabilities.

At step 206 of the present disclosure, the one or more hardware processors 104 are configured to determine a plurality of optimal sampling location on the farm in the specific region using the personalized crop protocol specific to the farm. The personalized crop protocol determines potential target pest, diseases and natural enemies that needs to be considered for the given farm and generates a potential spatio-temporal hot-spots layer. This spatio-temporal hot-spots layer changes over time based on parameters such as crop, growth stage, weather, abundance of natural enemies and/or the like. This spatio-temporal hot-spots layer derived from the personalized crop protocol determines the plurality of optimal sampling locations that needs to be visited for farm data collection at a given instance. The personalized crop protocol along with data on historical vulnerabilities to pests and diseases helps to map a target potential pest/diseases/natural enemies specific to a crop and its growth stage. As each pest and disease exhibit varying impact during different growth stages of the crops, weights are assigned to each pest/disease/natural enemies with respect to their potential impact on the crop and a pest/disease/natural enemies spatio-temporal hotspot layer is generated using the GIS environment. For instance, let $w_i$, represents the weight assigned to $i^{th}$ pest/disease/natural enemy. The spatio-temporal hot-spots layer highlights regions where the convergence of factors indicates a heightened likelihood of pest and disease presence. A plurality of hotspots are identified using the spatio-temporal hot-spots layer.

In an embodiment, the plurality of optimal sampling locations are determined based on a plurality of hotspot scores. In an embodiment, each hotspot score from the plurality of hotspots score corresponds to a hotspot from a plurality of hotspots identified by the spatio-temporal hot-spots layer. In an embodiment, a weighted overlay in the Geographic Information System (GIS) environment is created for hotspot score. It is assumed that $H(x, y)$ represent a weighted hotspot score at coordinate $(x, y)$, which is calculated as a sum of weighted factors as shown in equation (1) below:

$$H(x,y) = \sum i \, w_i \, * \, F_i(x,y) \, (1)$$

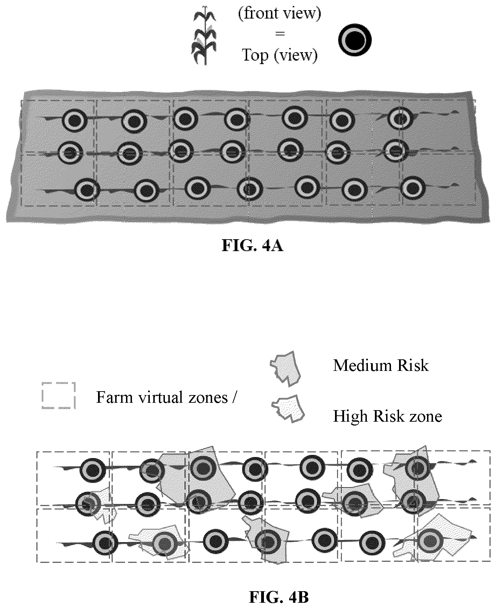

Here, $F_i(x, y)$ represents the contribution of factor $i$ at coordinate $(x, y)$. In an embodiment, the spatio-temporal hot-spots layer and thus the plurality of hotspots are dynamically updated based on the updates in the personalized crop protocol, inputs received from the one or more users on the farm and real-time sensor data acquired from the unmanned scouting vehicle. $H_{updated}(x, y)$ represents an updated hotspot score at coordinate $(x, y)$. The plurality of hotspots with high hotspot scores, as referenced from the knowledge graph database for a given crop and crop stage, are selected as the plurality of optimal sampling locations. FIGS. 4A and 4B show a top view of the farm illustrating identification of the plurality of hotspots for estimation of effective pest-disease severity score using a personalized crop protocol driven unmanned scouting vehicle, in accordance with some embodiments of the present disclosure. FIG. 4A shows the top view of the farm for estimation of effective pest-disease severity score using a personalized crop protocol driven unmanned scouting vehicle, in accordance with some embodiments of the present disclosure. As shown in FIG. 4A, the farm is divided into a plurality of grids or virtual zones. FIG. 4B shows the top view of the farm with a plurality of hotspots identified for estimation of effective pest-disease severity score using a personalized crop protocol driven unmanned scouting vehicle, in accordance with some embodiments of the present disclosure. As shown in FIG. 4B, the plurality of hotspots are identified and categorized as high-risk zone, medium risk zone, and lows risk zones in accordance with a high hotspot score, a medium hotspot score, and a low hotspot score respectively.

In an embodiment, each of the plurality of optimal sampling locations represents at least one of (i) a pest hotspot, (ii) a disease hotspot and (iii) a natural enemy hotspot. The plurality of optimal sampling locations are dynamically updated based on (i) one or more changes in the personalized crop protocol, (ii) a plurality of user inputs received on the farm and (iii) a plurality of real-time sensor data acquired from an unmanned scouting vehicle.

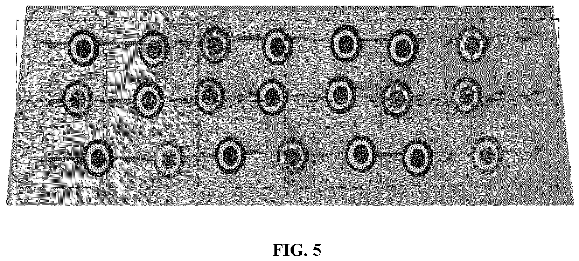

In an embodiment, at step 208 of the present disclosure, the one or more hardware processors 104 are configured to generate, a vehicle routing path protocol for an unmanned scouting vehicle based on the plurality of optimal sampling locations, a layout of the farm, information on potential growth of a plurality of plants projected by the personalized crop protocol and a plurality of available resources, using a reinforcement learning technique. The vehicle routing path protocol represents an optimal path required to be traversed by the unmanned scouting vehicle to reach each of the plurality of optimal sampling locations. FIG. 5 shows a top view of the farm illustrating the process of generation of the vehicle routing protocol for an unmanned scouting vehicle, in accordance with some embodiments of the present disclosure. In FIG. 5, a path to be taken by the unmanned scouting vehicle from a starting point to an ending point is shown. In other words, the vehicle routing protocol is generated that identifies most potential "starting point" for the "unmanned scouting vehicles", and a next node in the route and so on. This helps in finding a path to capture maximum points in a single journey to collect ground truth data to complement or validate the data and insights generated by multi-source data.

In an embodiment, the vehicle routing path protocol is generated using one or more routing algorithms such as Dijkstra's algorithm, A* algorithm and/or the like. If $P = \{p1, p2, ..., pn\}$ represent the plurality of optimal sampling locations and R represent a routing path connecting these locations, then $R$ is represented as provided in equation (2) below:

$$R = f\left(\begin{array}{l}\{p1, p2, ..., pn\}, field\ layout, topography, potential\ obstacles, \\ projected\ plant\ growth, and\ available\ resources\end{array}\right) (2)$$

In an embodiment, the reinforcement learning technique is used to optimize the unmanned scouting vehicle's path in real-time based on changing hotspot information and obstacles encountered. The reinforcement learning technique effectively combines hyper-localized data, personalized crop protocol, Geographic information system (GIS) analysis, weighting mechanisms, and routing algorithms to dynamically identify the plurality of hotspots and determine the plurality of optimal sampling locations while optimizing the path for the unmanned scouting vehicle. It enables precision data collection, adapts to real-time conditions, and maximizes resource utilization for efficient precision agriculture.

Referring to FIG. 2B, at step 210 of the present disclosure, the one or more hardware processors 104 are configured to collect a plurality of phenotyping characteristics of a plant at each plant position from a plurality of plant positions identified at each optimal sampling location from the plurality of optimal sampling locations on the optimal path traversed by the unmanned scouting vehicle. In the context of the present disclosure, the expressions 'plant positions' and 'plant nodes' can be interchangeably used throughout the description. The plurality of phenotyping characteristics of the plant is collected using a multi-degree-of-freedom mechanism on the unmanned scouting vehicle that adjusts one or more sensor positions with high precision allowing multi-angle data capture from a plurality of plant parts at a specific time instance. In an embodiment, the plurality of plant positions are dynamically identified using the personalized crop protocol and a knowledge graph.

Based on a multi-source-index based potential pest types (both good and bad bugs) and disease types, a risk summary of low, medium or high is generated. Output consists of the pest or disease type, potential density, and/or the impact on the plant. This advance knowledge can help the unmanned scouting vehicle to be ready with required tools or sensors for ground truthing or data collection. Based on received risk guidance, the unmanned scouting vehicle refers a knowledge graph, and looks at required phenotyping characteristics to be collected and corresponding sensors such as camera, ultrasound, and ground penetration radar to be used. The knowledge graph includes data on phenotypic traits that are indicators of pest and disease presence. For example, certain leaf discolorations or changes in leaf shape may indicate specific problems. The personalized crop protocol, based on real-time conditions, suggests which phenotypic traits are more likely to be affected by pests, diseases, or natural enemies at the given growth stage. By cross-referencing the knowledge graph and personalized crop protocol, relevant phenotype characteristics for data collection are selected, such as capturing images of the leafs undersides or spectroscopic data. Further, each phenotype characteristic might require specific sensors such as cameras, spectrometers, and/or the like to capture data accurately. The knowledge graph indicates which sensors are most suitable for detecting specific pest or disease symptoms. The personalized crop protocol, considering current weather, soil, and other conditions, helps in fine-tuning sensor positions to capture the desired data effectively. Based on the plurality of phenotype characteristic, and the knowledge graph, appropriate combination of sensors for each phenotype characteristic is selected and positions for each sensor is assigned such as such as lower side of the leaf, trunk, leaves' spectra, lower plant portion for video, and top of the plant for ultrasound. By combining the information from the knowledge graph and the personalized crop protocol, it can be intelligently determined where, how and using which sensors to collect data. This approach ensures that data collection efforts are focused on the most vulnerable areas and specific traits that are likely to provide meaningful insights for pest and disease management.

In other words, the knowledge graph provides insights into how pests, diseases, and natural enemies interact with different parts of the plant at specific growth stages. Data obtained from the personalized crop protocol reflects present situation of soil, weather, and farmer feedback and determines which growth stages are currently relevant for a crop in the farm. By combining this information, which plant positions are most susceptible to pest and disease activity at a present growth stage are identified.

The unmanned scouting vehicle also refer the challenges or the precautions to be taken while collecting the data such as appropriate illumination level, plant surface cleaning using air from small fan. The unmanned scouting vehicle also refer a potential height of the plant, at the stage since date of sowing, and then take a picture from different angle to compare and validate the required plant dimension. Accordingly, it adjusts a referred knowledge to find which "plant part(s)" to be scanned for data collection, at what levels, say leaves at the bottom side - nearer the root zone, or at the medium level, or top leaves, and/or the like. In other words, a knowledge graph can be referred to decide on the node priorities or the weights, and accordingly decide which nodes to scout first and thereafter to optimize the effort in terms of distance, and energy and time to collect the data.

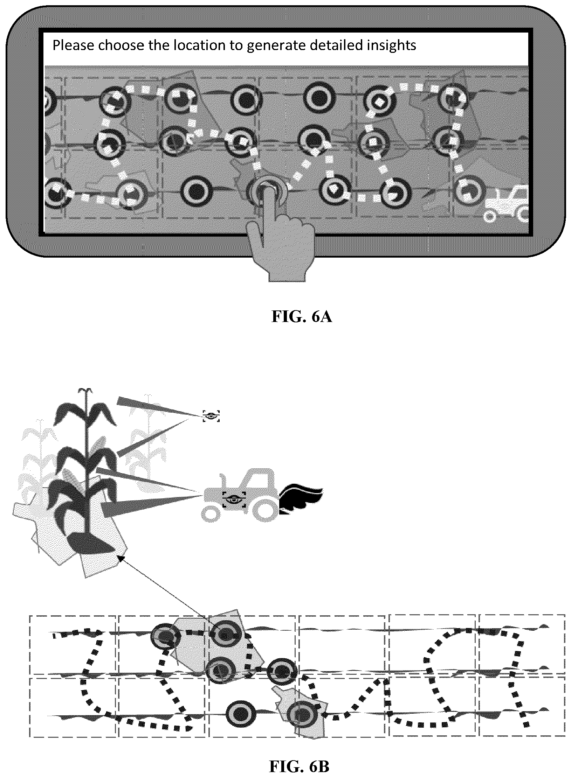

FIGS. 6A and 6B show a top view of the farm illustrating standardized and systematic way of data capture from precise plant positions using selected sensors and at the right time, in accordance with some embodiments of the present disclosure. FIG. 6A shows an optimal sampling location selected from the plurality of optimal sampling locations on the optimal path traversed by the unmanned scouting vehicle, in accordance with some embodiments of the present disclosure. As shown in FIG. 6A?, data obtained from the personalized crop protocol about the pest/disease/natural enemies' density/abundance at a given location is considered. Depending upon the potential type of pest, natural enemies, diseases and the associated crop growth stage, specific positions of the plant from where data needs to be collected are determined. FIG. 6B shows a plurality of plant positions to be traversed by the unmanned scouting vehicle for data collection, in accordance with some embodiments of the present disclosure. As shown in FIG. 6B, these specific positions of plants can be a plant location such as lower side of a leaf from young leaves, image of stem or trunk, spectra of leaves, video from lower portion of the plant, ultra-sound information from top of the plant, and/or the like. Depending upon various plant positions, a robotic arm or the multi-degree-of-freedom mechanism on the unmanned scouting vehicle adjusts the sensor positions with high precision, allowing for multi-angle data capture from various plant parts. This process is repeated at each plant location within the farm at the optimal path traversed by the unmanned vehicle.

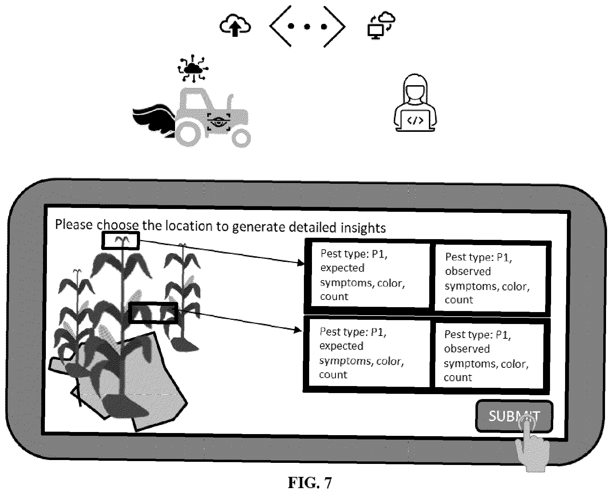

In an embodiment, the unmanned scouting vehicle possesses reinforcement learning ability. This means that the unmanned scouting vehicle has the capability to learn and adopt a localized data collection strategy based on learnings from the data captured by the unmanned scouting vehicle. In other words, the unmanned scouting vehicle can learn based on the collected data and decide if any action needs to be taken such as additional data collection or reduction in data-points. Moreover, the unmanned scouting vehicle can also consider any real-time user feedback received during the data capture to adjust in terms of plant positions and use of sensors. FIG. 7 shows a view of a computer or a handheld device for providing real-time user feedback, in accordance with some embodiments of the present disclosure. As shown in FIG. 7, data is acquired as per the personalized crop protocol as well as any incident feedback is shared by user such as a farm owner. Besides this, at the edge, post the data collection, patterns are generated, and the data is pre-processed to check if any additional insights can be generated regarding, if the data collected is sufficient for risk score calculation or additional data should be calculated. It also checks if there are any symptoms which are not categorized or identified by the existing model but are appearing frequently. It generates a brief note about that too. This can also lead to optimizing further data collection to see if route can be updated/reduced/extended to include more nodes.

In an embodiment, the quality of capturing data is an important aspect for performing accurate estimations.

Conventionally, a target an all-or-none approach is followed which means the unmanned scouting vehicle does not select which information is useful and which information is redundant or duplicate. As such, there is no control on repetitive information being captured, which causes an impact on various aspects on both edge and cloud where the data is used. The impact can be, but not limited to, over utilization of storage spaces, amount, frequency, and types of data being exchanged between the edge and the cloud, data transmitting charges, dimension of compute environments, network bandwidth requirement, upstream training of ML models with imbalanced datasets and inclusion of noise in data, and/or the like. Since most cloud resources involved in this process operate on costing model that depends on usage, this practice cannot remain sustainable unless an ever-incremental cost factor is shifted back to various stakeholders, amounting in increased financial load on one of the entities including farmers, farmer producer organizations, agri-input companies supporting these farmers, and/or the like. Conventionally, the focus has been on capturing all data that is available. This is not optimal. The present disclosure is striking a balance between what is considered a good data worth capturing in comparison to data that is redundant/duplicate and does not add any value to the current models or system, by making an intelligent pair of data exchanges and trained models between the edge devices (i.e., smart farm equipment) and cloud counterpart. In the present disclosure, this communication and understanding between the edge and cloud is also regularly enhanced with an automated feedback loop. In an embodiment, the unmanned scouting vehicle is equipped with data processing at edge to refrain from sharing redundant data to a data server. This means that the unmanned scouting vehicle is equipped with data processing at edge to avoid sharing redundant data to server and only send the output. For example, based on an image collected, it detects the disease, estimates its severity, and may only send the disease/-severity. This hybrid edge-cloud approach is optimal.

At step 212 of the present disclosure, the one or more hardware processors 104 are configured to compute a risk score for at least one of (i) the pest and (ii) the disease at the specific time instance at each plant position from the plurality of plant positions by processing the collected plurality of phenotyping characteristics of the plant using one or more models. In an embodiment, the one or more models may include but are not limited to one or more machine learning models, one or more data-based models, and/or the like. Data collected from multiple sensors is processed by using various machine learning, computer vision, and NLP algorithms. For each pest or disease or natural enemy that are represented by $i$, a risk score $R_i$ is computed based on sensor data, algorithms, and models. The risk score is determined at specific plant position levels as shown in equation (3) below:

$$ Ri = N_{i=0 \ to \ n} \ f(\sum P_i * w_i \ , \sum D_i * w_i \ ) \ (3) $$

Here, N is the number of pests species (P) and diseases (D) observed and the weights assigned to them based on the values referenced from the knowledge graph depending on their occurrence potential and potential impact at the crop health.

Further, at step 214 of the present disclosure, the one or more hardware processors 100 are configured to compute an aggregated plant risk score for the at least one of (i) the pest and (ii) the disease at the specific time instance in each plant from a plurality of plants with respect to crop stage. The aggregated plant risk score is the sum of the risk scores and a weightage associated with the at least one of (i) the pest and (ii) the disease at each plant position from the plurality of plant positions. The aggregated risk score is determined at plant levels. As each pest/disease/natural enemy has their impact/significance based on specific crop stage, the weightage is assigned to those pest/diseases/natural enemies. These weightages are determined dynamically and derived from knowledge graph. $w_i$ represents the weightage assigned to $i^{th}$ pest, disease, or natural enemy. In an embodiment, the knowledge graph helps in determining significance or impact of each pest and disease for each crop stage. For example, pest $X$ may have high impact during vegetative stage of the crop than pest $Y$, so high weightage will be given to $X$ than $Y$. Further, Pest $Y$ may be dangerous during crop flowering stage, so high weight will be given to pest $Y$ during flowering stage.

At step 216 of the present disclosure, the one or more hardware processors 104 are configured to estimate an effective pest-disease severity score for the farm based on a sum of the aggregated risk score for the at least one of (i) the pest and (ii) the disease at the specific time instance in each plant from a plurality of plants with respect to crop stage. The effective pest-disease severity score for the farm at a given time instance is estimated as shown in equation (4) below:

$$ EPS = \sum i \ w_i \ * \ R_i \ (4) $$

Here, *EPS* represents the effective pest-disease severity score.

The estimated *EPS* is utilized as feedback to optimize the plurality of optimal sampling locations and plant positions for data capture, enhance generation of the plurality of hotspots and selection process of the plurality of optimal sampling locations by incorporating the estimated pest-disease severity information and performing selection of the plurality of phenotyping characteristics of plants and corresponding sensors based on a current effective pest- severity score.

The estimated EPS values are integrated with spatial data to generate a map of effective pest-disease severity across the farm. $M(x, y)$ represents the effective pest-disease severity score value at coordinates $(x, y)$ on the map. The generated map is analysed to identify spatial patterns and zones of varying pest or disease severity and decision support to users such as farmers is provided by highlighting areas with high effective pest-disease severity that require immediate attention. In other words, the effective pest-disease severity score is cumulative score based on the good bug and bad bug pest intensity and occurrence. Based on the historical data of the pest or disease severity, a "1-2 or multi week ahead" pest or disease intensity forecast can be predicted too. The effective pest-disease severity score can also be used to "optimize ta Follow-up vehicle scouting protocol" for coming weeks, which can be shorter path or longer path than a simple rule based calculated path.

The written description describes the subject matter herein to enable any person skilled in the art to make and use the embodiments. The scope of the subject matter embodiments is defined herein and may include other modifications that occur to those skilled in the art. Such other modifications are intended to be within the scope of the present disclosure if they have similar elements that do not differ from the literal language of the embodiments or if they include equivalent elements with insubstantial differences from the literal language of the embodiments described herein.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated herein by the following claims.

**Claims**

**1.** A processor implemented method (200), comprising:

acquiring (202), via one or more hardware processors, a plurality of data pertaining to a farm in a specific region;

deriving (204), via the one or more hardware processors, a personalized crop protocol specific to the farm in the specific region using the plurality of data, wherein the personalized crop protocol captures dynamic interplay of one or more factors affecting a crop grown in the farm and suggests a precise sequence of practices required to be followed;

determining (206), via the one or more hardware processors, a plurality of optimal sampling locations on the farm in the specific region using the personalized crop protocol specific to the farm, wherein the plurality of optimal sampling locations are determined based on a plurality of hotspot scores, and wherein each of the plurality of optimal sampling locations represents at least one of (i) a pest hotspot, (ii) a disease hotspot and (iii) a natural enemies hotspot;

generating (208), via the one or more hardware processors, a vehicle routing path protocol for an unmanned scouting vehicle based on the plurality of optimal sampling locations, a layout of the farm, information on potential growth of a plurality of plants projected by the personalized crop protocol and a plurality of available resources, using a reinforcement learning technique, wherein the vehicle routing path protocol represents an optimal path required to be traversed by the unmanned scouting vehicle to reach each of the plurality of optimal sampling locations;

collecting (210), via the one or more hardware processors, a plurality of phenotyping characteristics of a plant at each plant position from a plurality of plant positions identified at each optimal sampling location from the plurality of optimal sampling locations on the optimal path traversed by the unmanned scouting vehicle, wherein the plurality of phenotyping characteristics of the plant are collected using a multi-degree-of-freedom mechanism on the unmanned scouting vehicle that adjusts one or more sensor positions with high precision allowing multi-angle data capture from a plurality of plant parts at a specific time instance;

computing (212), via the one or more hardware processors, a risk score for at least one of (i) the pest and (ii) the disease at the specific time instance at each plant position from the plurality of plant positions by processing the collected plurality of phenotyping characteristics of the plant using one or more models;

computing (214), via the one or more hardware processors, an aggregated plant risk score for the at least one of (i) the pest and (ii) the disease at the specific time instance in each plant from a plurality of plants with respect to crop stage, wherein the aggregated plant risk score is the sum of the risk scores and a weightage associated with the at least one of (i) the pest and (ii) the disease at each plant position from the plurality of plant positions; and

estimating (216), via the one or more hardware processors, an effective pest-disease severity score for the farm based on a sum of the aggregated risk score for the at least one of (i) the pest and (ii) the disease at the specific time instance in each plant from a plurality of plants with respect to crop stage.

2.  The processor implemented method as claimed in claim 1, wherein the plurality of optimal sampling locations are dynamically updated based on (i) one or more changes in the personalized crop protocol, (ii) a plurality of user inputs received on the farm and (iii) a plurality of real-time sensor data acquired from the unmanned scouting vehicle.

3.  The processor implemented method as claimed in claim 1, wherein the plurality of plant positions are dynamically identified using the personalized crop protocol and a knowledge graph.

4.  The processor implemented method as claimed in claim 1, wherein the unmanned scouting vehicle possesses reinforcement learning ability.

5.  The processor implemented method as claimed in claim 1, wherein the unmanned scouting vehicle is equipped with data processing at edge to refrain from sharing redundant data to a data server.

6.  A system (100), comprising:

    a memory (102) storing instructions;
    one or more communication interfaces (106);
    an unmanned scouting vehicle (112); and
    one or more hardware processors (104) coupled to the memory (102) via the one or more communication interfaces (106), wherein the one or more hardware processors (104) are configured by the instructions to:

    acquire a plurality of data pertaining to a farm in a specific region;
    derive a personalized crop protocol specific to the farm in the specific region using the plurality of data, wherein the personalized crop protocol captures dynamic interplay of one or more factors affecting a crop grown in the farm and suggests a precise sequence of practices required to be followed;

determine a plurality of optimal sampling location on the farm in the specific region using the personalized crop protocol specific to the farm , wherein the plurality of optimal sampling locations are determined based on a plurality of hotspot scores , and wherein each of the plurality of optimal sampling locations represents at least one of (i) a pest hotspot, (ii) a disease hotspot and (iii) a natural enemies hotspot;

generate a vehicle routing path protocol for an unmanned scouting vehicle based on the plurality of optimal sampling locations, a layout of the farm, information on potential growth of a plurality of plants projected by the personalized crop protocol and a plurality of available resources, using a reinforcement learning technique, wherein the vehicle routing path protocol represents an optimal path required to be traversed by the unmanned scouting vehicle to reach each of the plurality of optimal sampling locations;

collect a plurality of phenotyping characteristics of a plant at each plant position from a plurality of plant positions identified at each optimal sampling location from the plurality of optimal sampling locations on the optimal path reversed by the unmanned scouting vehicle, wherein the plurality of phenotyping characteristics of the plant are collected using a multi-degree-of-freedom mechanism on the unmanned scouting vehicle that adjusts one or more sensor positions with high precision allowing multi-angle data capture from a plurality of plant parts at a specific time instance;

compute a risk score for at least one of (i) the pest and (ii) the disease at the specific time instance at each plant position from the plurality of plant positions by processing the collected plurality of phenotyping characteristics of the plant using one or more models;

compute an aggregated plant risk score for the at least one of (i) a pest and (ii) a disease at the specific time instance in each plant from a plurality of plants with respect to crop stage, wherein the aggregated plant risk score is the sum of the risk scores and a weightage associated with the at least one of (i) the pest and (ii) the disease at each plant position from the plurality of plant positions; and

estimate an effective pest-disease severity score for the farm based on a sum of the aggregated risk score for the at least one of (i) the pest and (ii) the disease at the specific time instance in each plant from a plurality of plants with respect to crop stage.

7. The system as claimed in claim 6, wherein the plurality of optimal sampling locations are dynamically updated based on (i) one or more changes in the personalized crop protocol, (ii) a plurality of user inputs received on the farm and (iii) a plurality of real-time sensor data acquired from the unmanned scouting vehicle.

8. The system as claimed in claim 6, wherein the plurality of plant positions are dynamically identified using the personalized crop protocol and a knowledge graph.

9. The system as claimed in claim 6, wherein the unmanned scouting vehicle possesses reinforcement learning ability

10. The system as claimed in claim 6, wherein the unmanned scouting vehicle is equipped with data processing at edge to refrain from sharing redundant data to a data server.

11. One or more non-transitory machine-readable information storage mediums comprising one or more instructions which when executed by one or more hardware processors cause:

acquiring a plurality of data pertaining to a farm in a specific region;

deriving a personalized crop protocol specific to the farm in the specific region using the plurality of data, wherein the personalized crop protocol captures dynamic interplay of one or more factors affecting a crop grown in the farm and suggests a precise sequence of practices required to be followed;

determining a plurality of optimal sampling locations on the farm in the specific region using the personalized crop protocol specific to the farm, wherein the plurality of optimal sampling locations are determined based on a plurality of hotspot scores, and wherein each of the plurality of optimal sampling locations represents at least one of (i) a pest hotspot, (ii) a disease hotspot and (iii) a natural enemies hotspot;

generating a vehicle routing path protocol for an unmanned scouting vehicle based on the plurality of optimal sampling locations, a layout of the farm, information on potential growth of a plurality of plants projected by the personalized crop protocol and a plurality of available resources, using a reinforcement learning technique, wherein the vehicle routing path protocol represents an optimal path required to be traversed by the unmanned scouting vehicle to reach each of the plurality of optimal sampling locations;

collecting a plurality of phenotyping characteristics of a plant at each plant position from a plurality of plant positions identified at each optimal sampling location from the plurality of optimal sampling locations on the optimal path traversed by the unmanned scouting vehicle, wherein the plurality of phenotyping characteristics of the plant are collected using a multi-degree-of-freedom mechanism on the unmanned scouting vehicle that adjusts one or more sensor positions with high precision allowing multi-angle data capture from a plurality of plant parts at a specific time instance;

computing a risk score for at least one of (i) the pest and (ii) the disease at the specific time instance at each plant position from the plurality of plant positions by processing the collected plurality of phenotyping characteristics of the plant using one or more models;

computing an aggregated plant risk score for the at least one of (i) the pest and (ii) the disease at the specific time instance in each plant from a plurality of plants with respect to crop stage, wherein the aggregated plant risk score is the sum of the risk scores and a weightage associated with the at least one of (i) the pest and (ii) the disease at each plant position from the plurality of plant positions; and

estimating an effective pest-disease severity score for the farm based on a sum of the aggregated risk score for the at least one of (i) the pest and (ii) the disease at the specific time instance in each plant from a plurality of plants with respect to crop stage.

12. The one or more non-transitory machine-readable information storage mediums as claimed in claim 11, wherein the plurality of optimal sampling locations are dynamically updated based on (i) one or more changes in the personalized crop protocol, (ii) a plurality of user inputs received on the farm and (iii) a plurality of real-time sensor data acquired from the unmanned scouting vehicle.

13. The one or more non-transitory machine-readable information storage mediums as claimed in claim 11, wherein the plurality of plant positions are dynamically identified using the personalized crop protocol and a knowledge graph.

14. The one or more non-transitory machine-readable information storage mediums as claimed in claim 11, wherein the unmanned scouting vehicle possesses reinforcement learning ability.

15. The one or more non-transitory machine-readable information storage mediums as claimed in claim 11, wherein the unmanned scouting vehicle is equipped with data processing at edge to refrain from sharing redundant data to a data server.

System**100**

Processor(s) **104**

I/O Interface(s) **106**

Unmanned Scouting Vehicle **112**

Memory **102**

Database **108**

Modules **110**

**FIG. 1**

200

Acquiring via one or more hardware processors, a plurality of data pertaining to a farm in a specific region — 202

Deriving, via the one or more hardware processors, a personalized crop protocol specific to the farm in the specific region using the plurality of data, wherein the personalized crop protocol captures dynamic interplay of one or more factors affecting a crop grown in the farm and suggests a precise sequence of practices required to be followed — 204

Determining, via the one or more hardware processors, a plurality of optimal sampling location on the farm in the specific region using the personalized crop protocol specific to the farm, wherein the plurality of optimal sampling locations are determined based on a plurality of hotspot scores , and wherein each of the plurality of optimal sampling locations represents at least one of (i) a pest hotspot, (ii) a disease hotspot and (iii) a natural enemy hotspot — 206

Generating via the one or more hardware processors, a vehicle routing path protocol for an unmanned scouting vehicle based on the plurality of optimal locations, a layout of the farm, information on potential growth of a plurality of plants projected by the personalized crop protocol and a plurality of available resources, using a reinforcement learning technique, wherein the vehicle routing path protocol represents an optimal path required to be traversed by the unmanned scouting vehicle to reach each of the plurality of optimal locations — 208

**FIG. 2A**

Collecting, via the one or more hardware processors, a plurality of phenotyping characteristics of a plant at each plant position from a plurality of plant positions identified at each optimal location from the plurality of optimal locations on the optimal path reversed by the unmanned scouting vehicle, wherein the plurality of phenotyping characteristics of the plant are collected using a multi-degree-of-freedom mechanism on the unmanned scouting vehicle that adjusts one or more sensor positions with high precision allowing multi-angle data capture from a plurality of plant parts at a specific time instance

210

Computing, via the one or more hardware processors, a risk score for at least one of (i) the pest and (ii) the disease at the specific time instance at each plant position from the plurality of plant positions by processing the collected plurality of phenotyping characteristics of the plant using one or more models

212

Computing, via the one or more hardware processors, an aggregated plant risk score for the at least one of (i) the pest and (ii) the disease at the specific time instance in each plant from a plurality of plants with respect to crop stage, wherein the aggregated plant risk score is the sum of the risk scores and a weightage associated with the at least one of (i) the pest and (ii) the disease at each plant position from the plurality of plant positions

214

Estimating, via the one or more hardware processors, an effective pest-disease severity score for the farm based on a sum of the aggregated risk score for the at least one of (i) the pest and (ii) the disease at the specific time instance in each plant from a plurality of plants with respect to crop stage

216

**FIG. 2B**

**FIG. 3**

(front view)
=
Top (view)

**FIG. 4A**

Farm virtual zones /

Medium Risk

High Risk zone

**FIG. 4B**

**FIG. 5**

Please choose the location to generate detailed insights

**FIG. 6A**

**FIG. 6B**

Please choose the location to generate detailed insights

| | |
|---|---|
| Pest type: P1, expected symptoms, color, count | Pest type: P1, observed symptoms, color, count |
| Pest type: P1, expected symptoms, color, count | Pest type: P1, observed symptoms, color, count |

SUBMIT

**FIG. 7**

Europäisches Patentamt

European Patent Office

Office européen des brevets

# EUROPEAN SEARCH REPORT

Application Number

EP 24 21 3199

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2023/386132 A1 (CHEN STEVEN [US] ET AL) 30 November 2023 (2023-11-30) <br> * paragraphs [0010], [0039], [0042], [0137] - [0143], [0191] - [0192], [0195], [0204] * <br> * paragraphs [0134], [0137], [0156], [0174], [0195] * <br> * paragraphs [0029] - [0033] * | 1-15 | INV. G01N33/00 <br><br> ADD. A01B69/04 A01M1/02 |
| X | US 2023/079259 A1 (DELJKOVIC LEILA [NZ] ET AL) 16 March 2023 (2023-03-16) <br> * paragraphs [0051], [0146], [0195]; figures 1-2 * | 1,6,11 | |
| X | US 2023/292647 A1 (BAINBRIDGE JARED [GB] ET AL) 21 September 2023 (2023-09-21) <br> * claims 1-3, 11 * | 1,6,11 | |

| | |
|---|---|
| | TECHNICAL FIELDS SEARCHED (IPC) |
| | G01N A01B A01M |

The present search report has been drawn up for all claims

| Place of search | Date of completion of the search | Examiner |
|---|---|---|
| Munich | 25 March 2025 | Autran, Adrien |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 21 3199

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2025

| Patent document cited in search report | Publication date | Patent family member(s) | Publication date |
|---|---|---|---|
| US 2023386132 A1 | 30-11-2023 | US 2023386132 A1<br>US 2023410501 A1 | 30-11-2023<br>21-12-2023 |
| US 2023079259 A1 | 16-03-2023 | EP 4107512 A1<br>US 2023079259 A1<br>WO 2021167470 A1 | 28-12-2022<br>16-03-2023<br>26-08-2021 |
| US 2023292647 A1 | 21-09-2023 | EP 4150515 A1<br>GB 2598012 A<br>GB 2618896 A<br>US 2023292647 A1<br>WO 2021255458 A1<br>ZA 202300610 B | 22-03-2023<br>16-02-2022<br>22-11-2023<br>21-09-2023<br>23-12-2021<br>29-11-2023 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202321083027